# EUROPEAN PATENT APPLICATION

(11) **EP 1 400 810 A2**
(43) Date of publication of application: **24.03.2004**
(21) Application number: 03027612.5
(22) Date of filing: 07.04.2000
(51) Int. Cl.: G01N 33/74, A61P 19/10, A61K 31/00, A61K 45/06, A61K 31/663, A61K 31/138, A61K 31/4535

(54) **Estrogen receptors and bone disease**

(30) Priority: 09.04.1999 GB 9908235; 14.07.1999 US 143716 P
(62) Divisional of application: 00926864.0
(71) Applicant: KARO BIO AB, 141 57 Huddinge (SE)
(72) Inventor: The designation of the inventor has not yet been filed
(74) Representative: Dean, John Paul

(57) **Abstract**

This invention relates to estrogen receptors and bone. In particular the invention relates to an osteoporosis treatment or prevention composition comprising or consisting of an ERβ-selective antagonist.

## Description

This invention relates to the use of modulators for estrogen receptor beta (ERβ) in hormone replacement therapy for metabolic bone disease. In particular, the invention relates to the use of ERβ antagonists and partial agonists to increase cortical bone mineral content and cortical bone strength.

Estrogen deficiency is a major risk factor for development of osteoporosis in post-menopausal women. In particular, the condition is primarily caused by the severe decrease of serum estrogen levels after cessation of ovarian function. The absence of estrogen results in increased bone resorption and a progressive negative bone remodelling balance, leading to reduced trabecular and cortical bone mass and an increased risk of bone fracture.

Osteoporosis is generally preceded by a condition called "osteopenia". Osteopenia is considered to be a condition where the subject has a BMD value between 1 and 2.5 standard deviations (S.D.) below the young adult mean. In osteoporosis the BMD is more than 2.5 SD below the young adult mean. Osteoporosis is considered established when one or more bone fractures have occurred. In the present application, terms such as "treatment" or "prevention" or "minimising the risk of" of "osteoporosis" embrace treatment or prevention or minimising the risk of "osteopenia" or "established osteoporosis."

The absence of estrogen in post menopausal women results in an increase in bone turnover (Turner, R.T., *et al* (1994) *Endocr Rev* **15,** 275-300) and a negative bone remodelling balance, leading to bone loss and an increased fracture risk. The decrease in bone mass can be prevented by treatment with estrogens (Lindsay, R., *et al* (1976) *Lancet* **1,** 1038-1041). Although the bone preserving effect of estrogen replacement is indisputable (Turner *et al supra* (1994), the cellular mechanism of action for this hormone effect is unclear. Osteoblasts express estrogen receptors (Eriksen, E.F., *et al* (1988) *Science* **241,** 84-86, Komm, B.S., *et al* (1988) *Science* **241,** 81-84), suggesting a direct effect of estrogens on bone tissue. However, other, indirect effects of estrogens on bone cannot be excluded.

It has been reported that a man who was homozygous for an inactivating point mutation in the ERα gene demonstrated unfused growth plates and severe osteopenia (Smith, E.P., *et al* (1994) *N Engl J Med* **331**, 1056-1061). Based on this clinical finding it was assumed that ERα is important for growth plate closure and for adult bone metabolism at least in human males. In contrast, mice lacking a functional ERα had only minor skeletal abnormalities (Taki, M., *et al* (1997) *J Bone Miner Res* **12,** S460), suggesting that other mechanisms or receptors for estrogen action might be important during skeletal development of the mouse.

The presence of both estrogen receptors (ERα and β) in bone cells suggest that at least some estrogenic effects are directly acting on bone tissue.

The cloning of the novel estrogen receptor, ERβ, suggested that there may exist alternative mechanisms of action for estrogen (Kuiper, G.G., *et al* (1996) *Proc Natl Acad Sci U S A* **93**, 5925-5930). We and others have demonstrated that ERβ is expressed in growth plate chondrocytes and osteoblasts, indicating a possible role for ERβ in the regulation of longitudinal bone growth and/or adult bone metabolism (Onoe, Y., *et al* (1997) *Endocrinology* **138,** 4509-4512, Arts, J., Kuiper, G.G., *et al* (1997) *Endocrinology* **138**, 5067-5070, Vidal, O., *et al* (1999) *J Bone Miner Res* 14 923-29 , Nilsson, L.O., *et al* (1999) *J Clin Endocrinol Metab* **84,** 370-373; Windahl Bone **26** 117-121 (2000)). However, the physiological role of ERβ in the regulation of growth and bone metabolism is still unknown.

The molecular mechanisms of action for ERα versus ERβ have recently been investigated. ERα and ERβ have almost identical DNA-binding domains and studies *in vitro* have demonstrated that the two receptors have similar affinities for estrogenic compounds (Kuiper, G.G. *et al* (1996) *Proc Natl Acad Sci U S A* **93,** 5925-5930, Kuiper, G.G., *et al* (1997) *Endocrinology* **138,** 863-870, Tremblay, G.B., *et al* (1997) *Mol Endocrinol* **11**, 353-365). The amino-acid sequence of ERβ differs from ERα in the N- and C-terminal trans-activating regions. Therefore the transcriptional activation mediated by ERβ may be distinct from that of ERα (Paech, K., *et al* (1997) *Science* **277,** 1508-1510). Considering the great similarities in ligand- and DNA- binding specificity it has been speculated that a differential tissue distribution of estrogen receptors may be important for mediating tissue specific responses to estrogens (Kuiper, G.G., and Gustafsson, J.A. (1997) *FEBS Lett* **410**, 87-90). Thus, the unique trans-activating domains of the two receptor subtypes, in combination with differential tissue-distribution, or differential cell-type distribution within a tissue, could be important factors to determine the estrogen response in target tissues.

We have recently generated mice devoid of functional ERβ protein and reported that ERβ is essential for normal ovulation efficiency, but is not essential for female or male sexual development, fertility, or lactation (Krege, J.H., *et al* (1998) *Proc Natl Acad Sci U S A* **95,** 15677-15682).

Mice lacking a functional ERβ receptor protein were generated by gene targeting (Krege, J.H. *et al* Proc. Natl Acad. Sci, (1998), **95,** 15677-15682. Studies aiming to determine the physiological role of ERβ on bone structure and bone mineral content were conducted using dual X-ray absorptiometry (DXA) and Peripheral Quantitative Computerized Tomography (pQCT). These measurements were applied to male and female 4-week-old (pre-pubertal) and 12-week-old (adult) mice of the wild-type (+/+) or mutant (-/-) genotypes. In the pre-pubertal mice, body weights, length and mineral content of long bones were indistinguishable between wild-type and mutant mice of either sex. Surprisingly, in adult female ERβ -/- mice, the body weight was increased, the femoral bones were longer and had an increased bone mineral content (DXA) compared to wild-type female mice of the same age. In fact, the bones of female ERβ -/- were masculinized with regard to the parameters analysed. This bone phenotypic alteration was not restricted to long bones, but was also noted in vertebral and calvarial bones. In the tibial long bone, which has almost completed its longitudinal growth before the onset of puberty, the increase in bone mineral content was still observable in the mutant compared to wild-type female adult mice, showing that increased bone mineral content was independent of longitudinal bone growth. It is well known that ovariectomy in young growing rodents results in a decreased trabecular bone mineral density but also in an increased radial growth of the cortical bone. Both these effects are reversed by treatment with estrogen. The increase in bone mineral content in ERβ (-/-) females was not due to increased trabecular bone mineral density but to an increased cross sectional cortical bone area associated with a radial bone growth. It appears that the cortical bone in adult female ERβ-/- mice demonstrated a loss of feminization, indicating that ERβ is essential for the endogenous estrogen effect on cortical bone (periosteal growth) in female mice.

When pQCT analysis was employed to distinguish whether the observed effects of ERβ depletion were affecting either trabecular or cortical bone mineral density, the results surprisingly showed that the increased bone mineral density was restricted to the cortical bone, and that the trabecular volumetric bone mineral density was not affected. This is unlike the effect of estrogen depletion during menopause or following oophorectomy, when the over-whelming effect is on trabecular bone mineral density. Measurements of cortical bone parameters showed that the bones of the female ERβ -/- mice, in comparison to their wild-type littermates, exhibited an increased periosteal as well as endosteal circumference, leading to increased cortical bone area. This increased cortical cross-sectional bone area was computed to result in a corresponding 30% increase in bone strength parameters, i.e cortical cross-sectional moment of inertia and the cortical moment of resistance. The biomechanic properties of ERβ -/- female bones may be further verified using e.g bending and torsion tests.

WO97/09348 (Karo Bio AB) discloses the cloning of ERβ receptor described in Kuiper, G. 1996 above. WO99/05170 (Yale University) discloses the amino-acid sequence and nucleotide sequence of another form of the ERβ gene and related protein sequences. WO98/56812 (Karo Bio AB) discloses certain ERα and ERβ selective ligands. Barkhem, T. *et al* Molecular Pharmacology **54** 105 - 112 (1998) discloses the selectivity of certain compounds for different forms of the estrogen receptor. WO99/11760 (The Regents of the University of California) discloses methods of screening test compounds for differential ligand activation of ER-α and ERβ at AP1 sites.

According to one aspect of the invention, there is provided an osteoporosis treatment composition comprising an ERβ antagonist or partial agonist.

Preferably the osteoporosis treatment composition comprises an ERβ-selective antagonist or a partial agonist. Preferably, the ERβ antagonist or partial agonist has no or negligible ERα-antagonist activity. Preferred antagonists have a binding affinity of less than 80nM, more preferably less than 10 nM for ERβ. Most preferably, the ERβ antagonist has a binding affinity of 0.001 to 10nM for ERβ. Negligible ERα antagonist activity may be determined with reference to estradiol (E2). Greater than or equal to 50% agonism compared to E2 may be considered negligible ERα antagonist activity.

The osteoporosis treatment composition may also contain an estrogen, an estrogen agonist or a SERM (selective estrogen receptor modulator) such as tamoxifen, raloxifene, droloxifene and tamoxifen methiodide, which SERMs display estrogen agonist-like activity on bone tissues and serum lipids but which are estrogen antagonists in breast and uterus. In addition to the ERβ antagonists, the composition may contain a conventional osteoporonis or osteopenia active agent. For example, in certain embodiments, the methods and compositions of the present invention may use/comprise a bisphosphonate. Suitable bisphosphonates correspond to the chemical formula: 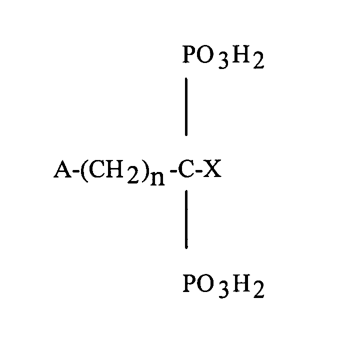 wherein n is an integer from 0 to about 7 and wherein A and X are independently selected from the group consisting of H, OH, halogen, NH2, SH, phenyl, C1-C30 alkyl, C3-C30 branched or cycloalkyl, C1-C30 substituted alkyl, C1-C10 alkyl substituted NH2, C3-C10 branched or cycloalkyl substituted NH2, C1-C10 dialkyl substituted NH2, C3-C10 branched or cycloalkyl disubstituted NH2, C1-C10 alkoxy, C1-C10 alkyl substituted thio, thiophenyl, halophenylthio, C1-C10 alkyl substituted phenyl, pyridyl, furanyl, pyrrolidinyl, imidazolyl, imidazopyridinyl, and benzyl, such that both A and X are not selected from H or OH when n is 0; or A and X are taken together with the carbon atom or atoms to which they are attached to form a C3-C10 ring.

In the foregoing chemical formula, the alkyl groups can be straight, branched, or cyclic, provided that sufficient atoms are selected for the chemical formula. The C1-C30 substituted alkyl can include a wide variety of substituents, nonlimiting examples which include those selected from the group consisting of phenyl, pyridyl, furanyl, pyrrolidinyl, imidazonyl, NH2, C1-C10 alkyl or dialkyl substituted NH2, OH, SH, and C1-C10 alkoxy.

The foregoing chemical formula is also intended to encompass complex carbocyclic, aromatic and hetero atom structures for the A and/or X substituents, non-limiting examples of which include naphthyl, quinolyl, isoquinolyl, adamantyl, and chlorophenylthio.

One class of structures which is useful includes those in which A is selected from the group consisting of H, OH, and halogen, X is selected from the group consisting of C1-C30 alkyl, C1-C30 substituted alkyl, halogen, and C1-C10 alkyl or phenyl substituted thio, and n is 0.

A subclass of structures includes those in which A is selected from the group consisting of H, OH, and C1, X is selected from the group consisting of C1-C30 alkyl, C1-C30 substituted alkyl, C1, and chlorophenylthio, and n is 0. An example of the subclass is when A is OH and X is a 3-aminopropyl moiety, and n is 0, so that the resulting compound is a 4-amino-1,-hydroxybutylidene-1,1-bisphosphonate, i.e. alendronate.

Pharmaceutically acceptable salts and derivatives of the bisphosphonates are also useful herein. Nonlimiting examples of salts include those selected from the group consisting alkali metal, alkaline metal, ammonium, and mono-, di, tri-, or tetra-C1-C30-alkyl-substituted ammonium. Preferred salts are those selected from the group consisting of sodium, potassium, calcium, magnesium, and ammonium salts. More preferred are sodium salts including mono and di and other higher sodium salts. Nonlimiting examples of derivatives include those selected from the group consisting of esters, hydrates, and amides. Hydrates can include whole number hydrates, i.e. monohydrates, dihydrates, trihydrates, etc., as well as fractional hydrates, such as for example, a hemi-pentahydrate (i.e. a 2.5 hydrate). Anhydrous forms of the bisphosphonates are also contemplated as within the scope of the present invention.

"Pharmaceutically acceptable" as used herein means that the salts and derivatives of the bisphosphonates have the same general pharmacological properties as the free acid form from which they are derived and are acceptable from a toxicity viewpoint.

It should be noted that the terms "bisphosphonate" and "bisphosphonates", as used herein in referring to the therapeutic agents of the present invention are meant to also encompass diphosphonates, biphosphonic acids, and diphosphonic acids, as well as salts and derivatives of these materials. The use of a specific nomenclature in referring to the bisphosphonate or bisphosphonates is not meant to limit the scope of the present invention, unless specifically indicated. Because of the mixed nomenclature currently in use by those or ordinary skill in the art, reference to a specific weight or percentage of a bisphosphonate compound in the present invention is on an acid active weight basis, unless indicated otherwise herein. For example, the phrase "about 70 mg of a bone resorption inhibiting bisphosphonate selected from the group consisting of alendronate, pharmaceutically acceptable salts thereof, and mixtures thereof, on an alendronic acid active weight basis" means that the amount of the bisphosphonate compound selected is calculated based on 70 mg of alendronic acid.

Nonlimiting examples of bisphosphonates useful herein include the following:
Alendronic acid, 4-amino-1-hydroxybutylidene-1,1-bisphosphonic acid.
Alendronate (also known as alendronate sodium or monosodium trihydrate), 4-amino-1-hydroxybutylidene-1,1-bisphosphonic acid monosodium trihydrate.
Alendronic acid and alendronate are described in U.S. Patents 4,922,007, to Kieczykowski *et al.,* issued May 1, 1990, and 5,019,651, to Kieczykowski, issued May 28, 1991, both of which are incorporated by reference herein in their entirety.
1,1-dichloromethylene-1,1-diphosphonic acid (clodronic acid), and the disodium salt (clodronate, Procter and Gamble), are described in Belgium Patent 672,205 (1966) and *J. Org. Chem* 32, 4111 (1967), both of which are incorporated by reference herein in their entirety.
1-hydroxy-3-(1-pyrrolidinyl)-propylidene-1,1-bisphosphonic acid (EB-1053).
1-hydroxyethane-1,1-diphosphonic acid (etidronic acid).
1-hydroxy-3-(N-methyl-N-pentylamino)propylidene-1,1-bisphosphonic acid, also known as BM-210955, Boehringer-Mannheim (ibandronate), is described in U.S. Patent No. 4,927,814, issued May 22, 1990, which is incorporated by reference herein in its entirety.
Cycloheptylaminomethylene-1,1-bisphosphonic acid, YM 175, Yamanouchi (incadronate, formerly known as cimadronate), as described in U.S. Patent 4,970,335, to Isomura et al., issued November 13, 1990, which is incorporated by reference herein in its entirety.
1-hydroxy-2-imidazo-(1,2-a)pyridin-3-yethylidene (minodronate).
6-amino-1-hydroxyhexylidene-1,1-bisphosphonic acid (neridronate).
3-(dimethylamino)-1-hydroxypropylidene-1,1-bisphosphonic acid (olpadronate).
3-amino-1-hydroxypropylidene-1,1-bisphosphonic acid (pamidronate).
[2-(2-pyridinyl)ethylidene]-1,1-bisphosphonic acid (piridronate) is described in U.S. Patent No. 4,761,406, which is incorporated by reference in its entirety.
1-hydroxy-2-(3-pyridinyl)-ethylidene-1,1-bisphosphonic acid (risedronate).
(4-chlorophenyl)thiomethane-1,1-disphosphonic acid (tiludronate) as described in U.S. Patent 4,876,248, to Breliere et al., October 24, 1989, which is incorporated by reference herein in its entirety.
1-hydroxy-2-(1H-imidazol-1-yl)ethylidene-1,1-bisphosphonic acid (zolendronate).

Preferred are bisphosphonates selected from the group consisting of alendronate, clodronate, etidronate, ibandronate, incadronate, minodronate, neridronate, risedronate, piridronate, pamidronate, tiludronate, zoledronate, pharmaceutically acceptable salts or esters thereof, and mixtures thereof.

More preferred is alendronate, ibandronate, risedronate, pharmaceutically acceptable salts or esters thereof, and mixtures thereof.

More preferred is alendronate, pharmaceutically acceptable salts thereof, and mixtures thereof.

Most preferred is alendronate monosodium trihydrate.

In other embodiments, other preferred salts are the sodium salt of ibandronate, and risedronate monosodium hemi-pentahydrate (i.e. the 2.5 hydrate of the monosodium salt).

It is recognized that mixtures of two or more of the bisphosphonate actives can be utilized.

The precise dosage of the bisphosphonate will vary with the dosing schedule, the particular bisphosphonate chosen, the age, size, sex and condition of the mammal or human, the nature and severity of the disorder to be treated, and other relevant medical and physical factors. Thus, a precise therapeutically effective amount cannot be specified in advance and can be readily determined by the caregiver or clinician. Appropriate amounts can be determined by routine experimentation from animal models and human clinical studies. Generally, an appropriate amount of bisphosphonate is chosen to obtain a bone resorption inhibiting effect, i.e. a bone resorption inhibiting amount of the nitrogen-containing bisphosphonate is administered. For humans, an effective oral dose of nitrogen-containing bisphosphonate is typically from about 1.5 to about 6000 µg/kg body weight and preferably about 10 to about 2000 µg/kg of body weight.

For the bisphosphonate, alendronate monosodium trihydrate, common human doses which are administered are generally in the range of about 2 mg/day to about 40 mg/day, preferably about 5 mg/day to about 40 mg/day. In the U.S. presently approved dosages for alendronate monosodium trihydrate are 5 mg/day for preventing osteoporosis, 10 mg/day for treating osteoporosis, and 40 mg/day for treating Paget's disease.

In alternative dosing regimens, the bisphosphonate can be administered at intervals other than daily, for example once-weekly dosing, twice-weekly dosing, biweekly dosing, and twice-monthly dosing. In such dosing regimens, appropriate multiples of the bisphosphonate dosage would be administered. For example, in a once weekly dosing regimen, alendronate monosodium trihydrate would be administered at dosages of 35 mg/week or 70 mg/week in lieu of seven consecutive daily dosages of 5 mg or 10 mg.

For ibandronate the unit dosage can comprise from about 2.5 mg to about 200 mg, on an ibandronic acid active weight basis, i.e. calculated on the basis of the corresponding acid. Examples of daily oral dosages comprise about 2.5 mg, 3.5 mg, 5 mg, 7.5 mg, and 10 mg. Examples of weekly oral dosages include a unit dosage which is useful for inhibiting bone resorption, and treating and preventing osteoporosis selected from the group consisting of about 25 mg, 30 mg, 35 mg, 40 mg, 45 mg, or 50 mg.

For risedronate the unit dosage can comprise from about 2.5 mg to about 200mg, on a risedronic acid active weight basis, i.e. calculated on the basis of the corresponding acid. Examples of daily oral dosages comprise about 2.5 mg, 3.5 mg, 5 mg (an exemplary osteoporosis daily dosage), 7.5 mg, and 10 mg, and 30 mg (an exemplary Paget's disease daily dosage). Examples of weekly oral dosages include a unit dosage which is useful for inhibiting bone resorption, and treating and preventing osteoporosis selected from the group consisting of about 25 mg, 30 mg, 35 mg, 40 mg, 45 mg, or 50 mg.

The pharmaceutical compositions herein may comprise from about 1 mg to about 100 mg of bisphosphonate, preferably from about 2 mg to 70 mg, and more preferably from about 5 mg to about 70, on a bisphosphonic acid basis. For the bisphosphonate alendronate monosodium trihydrate, the pharmaceutical compositions useful herein may comprise about 2.5 mg, 5 mg, 10 mg, 35, mg, 40 mg, or 70 mg of the active on an alendronic acid active weight basis.

See also, U.S. Patent 4,610,077, to Rosini et al., issued November 4, 1986; U.S. Patent 5,358,941, to Bechard et al., issued October25, 1994; and PCT application number WO 99/04773, to Daifotis et al., published February 4, 1999; all of which are incorporated by reference herein in their entirety.

Further embodiments of the methods and compositions of the present invention can comprise additional bone agents useful for inhibiting bone resorption and providing the desired therapeutic benefits of the invention. Examples of such agents include those selected from the group consisting of calcitonin, estrogens,progesterone, androgens, calcium supplements, fluoride, growth hormone secretagogues, vitamin D analogues, and selective estrogen receptor modulators. The calcitonins useful herein can be from human or nonhuman sources, e.g. salmon calcitonin. Nonlimiting examples of estrogens include estradiol. Nonlimiting examples of selective estrogen receptor modulators include raloxifene, iodoxifene, and tamoxifene. Growth horomone secretagogues are described in U.S. Patent No. 5,536,716, to Chen et al., issued July 16, 1996, which is incorporated by reference herein in its entirety.

According to another aspect of the invention there is provided the use of an ERβ antagonist in the preparation of a pharmaceutical for the treatment of osteoporosis; reduction of bone resorption in an adult mammal; stabilising or increasing cortical bone mass in an adult mammal; or reducing the risk of bone fracture in an adult mammal.

According to a further aspect of the invention, there is provided a method of treating osteoporosis; reducing bone resorption; increasing or stabilising bone trabecular mass; or increasing or stabilising cortical bone mass in an adult mammal; any of the such methods comprising supplying an ERβ antagonist to the mammal.

According to a further aspect of the invention, there is provided a method of selecting compounds for the treatment of osteoporosis, the method comprising selecting a compound on the basis of its ability to antagonise agonist-dependent ERβ activity. Preferably, the method of selecting compounds for the treatment of osteoporosis then includes a further step of testing of the compound in relation to bone-related parameters selected from bone resorption trabecular mass, cortical mass and reduction of fracture risk. The invention also provides chemical compounds useful for the treatment of osteoporosis determined by such a method of selecting compounds.

Pharmaceutical compositions of this invention comprise any of the compounds of the present invention, and pharmaceutically acceptable salts thereof, with any pharmaceutically acceptable carrier, adjuvant or vehicle. Pharmaceutically acceptable carriers, adjuvants and vehicles that may be used in the pharmaceutical compositions of this invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

The pharmaceutical compositions of this invention may be administered orally, parenterally, by inhalation spray, topically, rectally, nasally, buccally, vaginally or via an implanted reservoir. The pharmaceutical compositions of this invention may contain any conventional non-toxic pharmaceutically-acceptable carriers, adjuvants or vehicles. The term parenteral as used herein includes subcutaneous, intracutaneous, intravenous, intramuscular, intra-articular, intrasynovial, intrasternal, intrathecal, intralesional and intracranial injection or infusion techniques.

The pharmaceutical compositions may be in the form of a sterile injectable preparation, for example, as a sterile injectable aqueous or oleaginous suspension. This suspension may be formulated according to techniques known in the art using suitable dispersing or wetting agents (such as, for example, Tween 80) and suspending agents. The sterile injectable preparation may also be a sterile injectable solution or suspension in a non-toxic parenterally-acceptable diluent or solvent, for example, as a solution in 1,3-butanediol. Among the acceptable vehicles and solvents that may be employed are mannitol, water, Ringer's solution and isotonic sodium chloride solution. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant such as Ph. Helv or a similar alcohol.

The pharmaceutical compositions of this invention may be orally administered in any orally acceptable dosage form including, but not limited to, capsules, tablets, and aqueous suspensions and solutions. In the case of tablets for oral use, carriers which are commonly used include lactose and corn starch. Lubricating agents, such as magnesium stearate, are also typically added. For oral administration in a capsule form, useful diluents include lactose and dried corn starch. When aqueous suspensions are administered orally, the active ingredient is combined with emulsifying and suspending agents. If desired, certain sweetening and/or flavoring and/or colouring agents may be added.

As the skilled artisan will appreciate, lower or higher doses than those recited above may be required. Specific dosage and treatment regimens for any particular patient will depend upon a variety of factors, including the activity of the specific compound employed, the age, body weight, general health status, sex, diet, time of administration, rate of excretion, drug combination, the severity and course of the disease, the patient's disposition to the disease and the judgment of the treating physician.

Methods and compositions in accordance with the invention will now be described with reference to the accompanying drawings, Figures 1 to 2, in which:
Fig. 1 shows DXA measurements of bone parameters of excised tibias; and
Fig. 2 is a DXA scan of female (top) tibia (mid) and vertebrae (bottom) from ERP -/- and wildtype (WT) mice.

In the following examples the methods set out below were used:

### Animals

The animals were maintained under standardized environmental conditions, with free access to food and water. Genotyping of tail DNA was performed at 4-5 weeks of age using PCR with partly different primers than previously described (Krege, J.H., *et al* (1998) *supra.* The primers used were one primer in intron 2 (βNHD4-25; 5,-AGAATGTTGCACTGCCCCTGCTGC-3,), one in intron 3 (C1wt-27; 5-GGAGTAGAAACAAGCAATCCAGACATC-3,) and one in the Neo cassette (Neo-25; 5,-GCAGCCTCTGTTCCACATACACTTC-3,). The PCR program used was: 95°C 2 min, (95°C 30 s, 64°C 1 min, 72°C 1 min) for 30 cycles, and 72°C 7 min. A 650 bp product (βNHD4-25 and C1wt-27) was amplified for the homozygous wild-type (+/+) mice, a 450bp (βNHD4-25 and Neo-25) product was amplified for the homozygous mutant (-/-) mice and both bands were amplified for the heterozygous (+/-) mice.

### Histological examination

Right femora were fixed in 4% paraformaldehyde, embedded in paraffin, sectioned and stained with Alcianblue/Van Gieson. The width of the growth plate was measured using an image-processing system (Easy Image, Bergströms Instrument, Stockholm, Sweden) coupled to a microscope. The average of 30 growth plate measurements (3 sections, 10 measurements/section) was calculated for each femur.

### Dual X-Ray Absorptiometry (DXA)

Areal Bone mineral density (Areal BMD; BMC/cm²) and bone mineral content (BMC) were measured with the Norland pDEXA Sabre (Fort Atkinson, WI) and the Sabre Research software (3.6).

*In vivo* measurements of animals were performed in order to determine total body, spine and cranium BMC (medium resolution scan with line spacing set at 0.05 cm). Three mice were analyzed at a time. A mouse which was sacrificed at the beginning of the experiment was included in all the scans as an internal standard in order to avoid inter-scan variations.

*Ex vivo* measurements of the left femur and tibiae and vertebrae L5 were performed on excised bones placed on a 1 cm thick Plexiglas table. All bones compared were measured in the same scan (high resolution scan with line spacing set at 0.01 cm).

### Peripheral Quantitative Computerized Tomography (pQCT)

Computerized tomography was performed with the Stratec pQCT XCT Research M (Norland, software version 5.4B) operating at a resolution of 70 µm (Rosen, *H.N., et al* (1995) *Calcif Tissue Int* **57,** 35-39).

*Mid-diaphyseal pQCT scans* of femora and tibias were performed to determine the cortical volumetric bone mineral density (volumetric BMD), the cortical cross sectional area, the periosteal circumference, the endosteal circumference, the moment of resistance and the cross sectional moment of inertia. The mid-diaphyseal region of femora and tibias in mice contains only cortical bone.

*Metaphyseal pQCT scans* of left femora and tibias were performed to measure trabecular volumetric BMD. The scan was positioned in the metaphysis at a distance from the distal growth plate corresponding to 4% of the total length of the femur (an area containing cortical as well as trabecular bone). The trabecular bone region was defined by setting an inner threshold to 400 mg/mm³. The inter assay coefficients of variation (CV) for the pQCT measurements were less than 2%.

It should be emphasized that the DXA technique gives the areal BMD whereas the pQCT gives the real/volumetric BMD. Thus, the DXA technique gives the mineral content per area and not per volume. Therefore, a factor regulating the outer dimensions of a bone, will affect the areal BMD (DXA) but not the volumetric BMD (pQCT).

### Body Weight and Dimensions of Bones

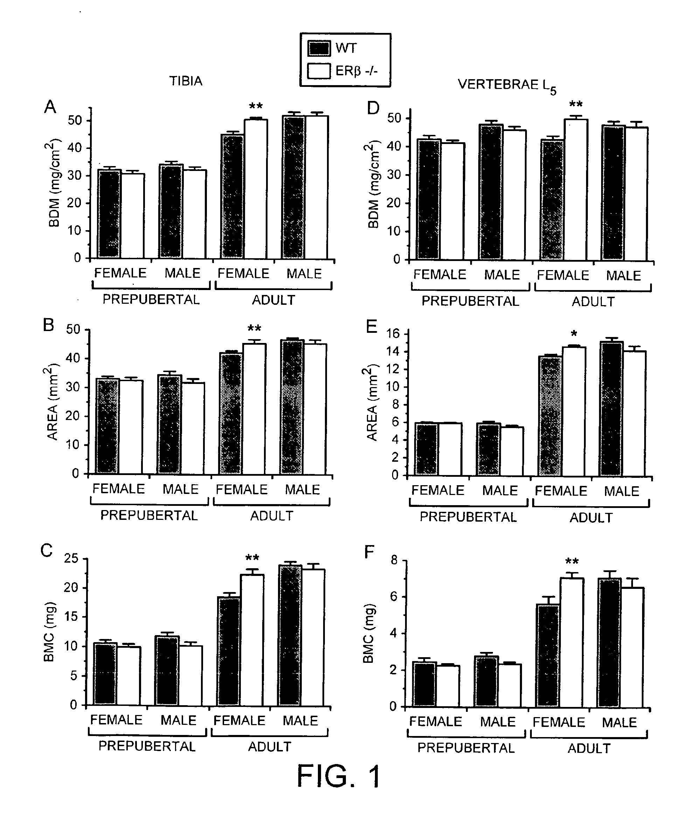

ERβ-/- and wild type mice were analyzed at 4 (Prepubertal) and 11 (Adult) weeks of age. The body weight, and length of the long bones were unchanged in prepubertal ERβ-/- mice compared with wt mice. No significant gender difference was seen between prepubertal wt females and wt males (t-test). However, adult male mice were, as expected, heavier and had longer tibias and femora than adult female mice (The results are shown in Table 1 and in Fig 1, t-test).

**Table 1**

| **Body Weight and Dimensions of Bones** | | | | | |
|---|---|---|---|---|---|
| | | **Female** | | **Male** | |
| | | WT | ERβ-/- | WT | ERβ-/- |
| **Prepubertal mice** | Body weight (g) | 14.8±0.5 | 14.7±0.6 | 16.7±0.6 | 16.1±0.4 |
| | Tibia length (mm) | 16.6±0.07 | 16.7±0.1 | 16.5±0.14 | 16.4±0.04 |
| | Femur length (mm) | 11.2±0.08 | 11.3±0.08 | 11.4±0.14 | 11.6±0.10 |
| **Adult mice** | Body weight (g) | 19.9±0.4 | 23.6± 1.1 ** | 25.9±0.6 | 26.8±1.7 |
| | Tibia length (mm) | 17.6±0.11 | 17.9±0.11 | 18.2±0.04 | 18.0±0.16 |
| | Femur length (mm) | 14.6±0.13 | 15.2±0.11** | 15.0±0.09 | 15.2±0.20 |
| | Femur width (mm) | 1.6±0.19 | 1.83±0.13* | | |
| | Femur growth plate width (mm) | 123±8.1 | 106±5.6 | 99±6.1 | 106±11.1 |
| | Cortical thickness (mm) | 0.47±0.06 | 0.5±0.07 | | |
| | Growth plate height (µm) | 123± | 106±5.6 | | |
| | Growth plate volume density | 0.32±0.04 | 0.33±0.07 | | |

| | | | | | |
|---|---|---|---|---|---|
| 4 week old mice are indicated as prepubertal mice (n=6 for female WT and ER-/- mice; n=7 for male WT and ERβ -/- mice). 11 week old mice are indicated as adult mice (n=6/7 for female WT and ERβ -/- mice; n=5 for male WT and ERβ-/- mice). Femur growth plate width was measured in the distal femur. Values are given as means ±SEM. ^{*} p< 0.05, ^{**}p< 0.01 versus wild type (WT). | | | | | |

Specifically in the experiments illustrated by Fig 1 the area BMD (A, D), bone area (B, E) and BMC (C, F) of excised tibias (A-C) and vertebrae L5 (D-F) were measured using DXA technique as described in Methods. 4 week old mice are indicated as prepubertal 1 mice (n=6 for female WT and ERβ-/- mice; n=7 for male WT and ERβ-/- mice). 11 week old mice are indicated as adult mice (n=7 for female WT and ERβ-/- mice; n=5 for male WT and ERβ-/- mice). Values are given as means±SEM. ^{*} p< 0,05, ^{**}p< 0,01 versus wild type (WT).

Surprisingly, the adult female ERβ-/- mice were heavier and had longer femora than adult female wt mice (Table 1).

A disproportional longitudinal bone growth, with a 4% increase in tibial and 30 % increase in femoral length, was seen from 4 weeks of age to 11 weeks of age in female wt mice (Table 1). Most likely this difference is due to the fact that most of the tibial growth occurs before puberty, whereas the femur demonstrates a significant pubertal/adult growthspurt. This observation provides a possible explanation to the finding of the present study that femoral but not tibial length is increased in ERβ deficient female mice and furthermore suggests that ERβ plays a repressive role during pubertal growth of murine females.

### Total Bone Mineral Content and Bone Mineral Content of Individual Bones

The bones from the ERβ-/- mice were analyzed in detail using DXA and pQCT techniques.

The whole body BMC was increased in adult female but not in adult male ERβ-/- mice compared with wt mice as measured using DXA. The results are shown in Table 2.

**Table 2**

| **DXA Measurements of Bone Mineral Content *in vivo*** | | | | | | |
|---|---|---|---|---|---|---|
| | | | **Female** | | **Male** | |
| | | | WT | ERβ-/- | WT | ERβ-/- |
| **Prepubertal mice** | **Total body** | Bone mineral content (mg) | 367±18 | 334±11 | 418±13 | 393±17 |
| | **Cranium** | Bone mineral content (mg) | 140±4.0 | 123±4.1* | 145±3.4 | 138±3.8 |
| **Adult mice** | **Total body** | Bone mineral content (mg) | 507±15 | 596±15** | 603±20 | 626±34 |
| | **Cranium** | Bone mineral content (mg) | 201±2.9 | 234±5.9^{**} | 204±4.0 | 215±6.1 |
| | **Spine** | Bone mineral content (mg) | 70±4.3 | 92±5.9** | 103±11 | 94±8.6 |

| | | | | | | |
|---|---|---|---|---|---|---|
| 4 week old mice are indicated as prepubertal mice (n=6 for female WT and ERβ -/- mice; n=7 for male WT and ERβ -/- mice). 11 week old mice are indicated as adult mice (n=7 for female WT and ERβ -/- mice; n=5 for male WT and ERβ -/- mice). Values are given as means ± SEM. ^{*} p< 0.05, ^{**}p< 0.01 versus wild type (WT) | | | | | | |

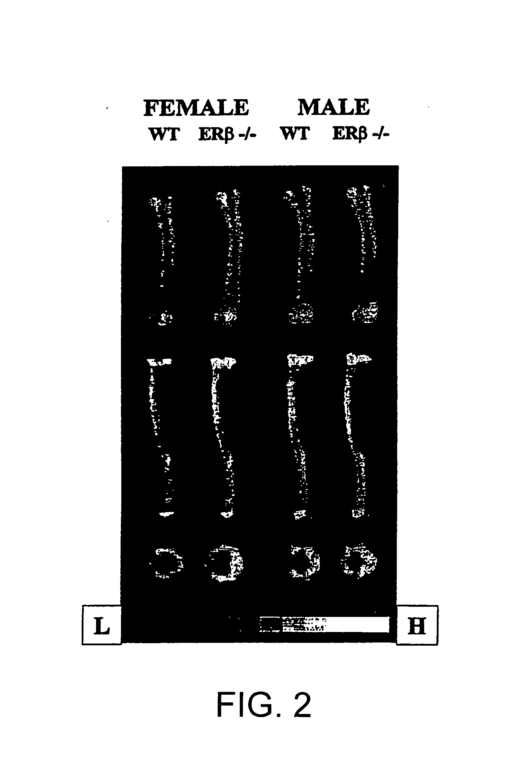

Actually, the gender difference seen in adult wt mice, with higher total BMC in males compared with females, was not seen in adult ERβ-/- mice (t-test). The effect on individual bones was also studied using DXA technique. The BMC of cranium, spine, vertebrae L5, tibia and femur were increased in adult female ERβ-/- mice compared with WT mice (Cranium 16%, spine 31 %, vertebrae L5 24%, tibia 20% and femur 27% over wt; Table 2, Fig 1-2). In Fig. 2 the relative areal BMD is indicated. (bottom; H= high density and L= low density). WT male mice had higher BMC than wt females in the spine, the vertebrae L5 and the tibia (Fig 1 and Table 2, t-test). This gender difference was not seen in ERβ-/mice. No difference in total-, spine-, tibial-and vertebral- BMC was seen between ERβ-/and wt in prepubertal male and female mice or in adult male mice.

The increase in tibial (20% over wt) and vertebral (24% over wt) BMC in adult female ERβ-/- mice was a result of both an increased bone area (Tibia 8%, vertebrae L5 11 % over wt) and an increased areal BMD (Tibia 11 %, vertebrae L5 16% over wt; Fig 1).

### Trabecular Bone Mineral Density

Trabecular volumetric BMD was measured in the metaphysis of the distal femur and proximal tibia using the pQCT technique. As seen in Table 3, adult but not prepubertal female mice had significantly lower trabecular volumetric BMD than male mice.

**Table 3**

| **Trabecular volumetric BMD and Cortical Bone Parameters as Measured using pQCT** | | | | | | |
|---|---|---|---|---|---|---|
| | | | **Female** | | **Male** | |
| | | | WT | ERβ-/- | WT | ERβ-/- |
| **Prepubertal mice** | **Tibia** | Trabecular density (mg/mm³) | 0.196±0.007 | 0.191±0.012 | 0.215±0.016 | 0.218±0.017 |
| | | Cortical density (mg/mm³) | 0.938±0.010 | 0.929±0.015 | 0.951±0.007 | 0.938±0.003 |
| | | Cortical area (mm²) | 0.48±0.03 | 0.46±0.02 | 0.52±0.02 | 0.54±0.01 |
| | | Cortical bone mineral content (mg/mm) | 0.46±0.03 | 0.43±0.03 | 0.49±0.02 | 0.50±0.01 |
| **Adult mice** | **Tibia** | Trabecular density (mg/mm³) | 0.282±0.011 | 0.299±0.009 | 0.331±0.006 | 0.328±0.007 |
| | | Cortical density (mg/mm³) | 1.130±0.010 | 1.172±0.004** | 1.168±0.011 | 1.173±0.015 |
| | | Cortical area (mm²) | 0.67±0.01 | 0.76±0.02^{**} | 0.85±0.03 | 0.83±0.03 |
| | | Cortical bone mineral content (mg/mm) | 0.75±0.02 | 0.89±0.02^{**} | 0.99±0.03 | 0.97±0.04 |
| | **Femur** | Trabecular density (mg/mm³) | 0.287±0.007 | 0.274±0.010 | 0.316±0.010 | 0.309±0.014 |
| | | Cortical density (mg/mm³) | 1.147±0.011 | 1.182±0.008* | 1.156±0.020 | 1.166±0.009 |
| | | Cortical area (mm²) | 0.80±0.02 | 0.91±0.03^{**} | 0.97±0.03 | 0.99±0.05 |
| | | Cortical bone mineral content (mg/mm) | 0.91±0.03 | 1.07±0.04** | 1.12±0.04 | 1.15±0.06 |

| | | | | | | |
|---|---|---|---|---|---|---|
| 4 week old mice are indicated as prepubertal mice (n=6 for female WT and ERβ -/- mice; n=7 for male WT and ERβ -/- mice). 11 week old mice are indicated as adult mice (n=7 for female WT and ERβ -/- mice; n=5 for male WT and ERβ -/- mice). Values are given as means ± SEM. ^{*} p< 0.05, ^{**}p< 0.01 versus wild type (WT) | | | | | | |

In contrast to the total BMC of tibia and femur no effect was seen on the trabecular volumetric BMD in adult female ERβ-/- mice compared with wt mice. Thus, the increased BMC in female ERβ -/- mice was not caused by an increased trabecular volumetric BMD.

### Cortical Bone Parameters

As the trabecular volumetric BMD was not changed the cortical bone parameters were studied in detail. Cortical bone parameters were investigated by mid-diaphyseal pQCT sections. The cortical BMCs of the mid-diaphyseal section of tibia and femur were increased in adult female ERβ-/- mice compared with wt (Tibia 19%, Femur 18% over wt) and this increase was mainly due to an increased cross-sectional bone area (Tibia 13%, Femur 14% over wt) and to a lesser extent an increased volumetric BMD (Tibia 4%, Femur 3% over wt; Table 3). As seen from Table 4, the increase in cross sectional area was associated with an increase of the periosteal circumference as well as the endosteal circumference.

**Table 4**

| **Mid-Diaphyseal Dimensions in Adult mice as Measured using pQCT** | | | | | |
|---|---|---|---|---|---|
| | | **Female** | | **Male** | |
| | | WT | ERβ-/- | WT | ERβ-/- |
| **Tibia** | Cortical periosteal circumference (mm) | 4.04±0.04 | 4.34±0.05** | 4.51±0.08 | 4.46±0.12 |
| | Cortical endosteal circumference (mm) | 2.82±0.05 | 3.05±0.03** | 3.12±0.07 | 3.09±0.11 |
| | Cortical cross sectional moment of inertia (mm⁴) | 0.17±0.01 | 0.24±0.02^{**} | 0.29±0.02 | 0.29±0.03 |
| | Cortical moment of resistance (mm³) | 0.23±0.01 | 0.29±0.01** | 0.32±0.02 | 0.30±0.02 |
| **Femur** | Cortical periosteal circumference (mm) | 4.93±0.07 | 5.28±0.06^{**} | 5.52±0.16 | 5.51±0.10 |
| | Cortical endosteal circumference (mm) | 3.78±0.08 | 4.06±0.06* | 4.28±0.18 | 4.24±0.08 |
| | Cortical cross sectional moment of inertia (mm⁴) | 0.33±0.02 | 0.46±0.02** | 0.54±0.05 | 0.56±0.05 |
| | Cortical moment of resistance (mm³) | 0.36±0.01 | 0.46±0.02^{**} | 0.49±0.03 | 0.50±0.03 |

| | | | | | |
|---|---|---|---|---|---|
| n=7 for female WT and ERβ-/- mice; n=5 for male WT and ERβ-/- mice. Values are given as means ± SEM. ^{*} p< 0.05, **p<0.01 versus wild type (WT) | | | | | |

The changes in the size and position of the cross-sectional cortical bone area in adult female ERβ-/- mice resulted in a large increase of both the cortical cross sectional moment of inertia (Tibia 41%, Femur 39% over wt) and the cortical moment of resistance (Tibia 26%, Femur 28% over wt; Table 4). There was a gender difference for all of the cortical bone parameters between adult female wt and male wt mice. However, no gender difference was seen for these parameters between female ERβ-/- mice and male ERβ-/mice.

### Effect of ovariectomy in adult female mice

Female ERβ -/- mice were ovariectomised (ovx) at 13 weeks of age. The bone of the animals were analyzed 8 weeks later. Ovx decreased the uterine weight in both ERβ -/and wt mice, demonstrating that the ovx was complete (Table 5). Ovx decreased the BMC to the same extent in ERβ -/- mice as in wt mice (Table 5). This decrease in BMC was associated with a large decrease in trabecular volumetric BMD and a small decrease in cortical BMC.

**Table 5**

| **Effect of ovariectomy in adult female mice** | | | |
|---|---|---|---|
| | | Female | |
| | | WT | ERβ -/- |
| **Uterine Weight** | | -82±3%** | -80 ±2%** |
| **Cranium** | Total BMC | 09.0±4% | -7.9 ±2.6% |
| **Vertebrae L**_{**5**} | Total BMC | -21 ±3% | -27 ±6% |
| **Tibia** | Total BMC | -20 ±1%* | -26±4%** |
| **Femur** | Total BMC | -18 ±2%* | -17±4* |
| | Trabecular density | -36 ±5% | -27 ±5%** |
| | Cortical BMC | -18 ±3** | -21 ±3** |
| | Cortical density | -6 ±0.9%** | -4 ±0.4%** |
| | Cortical area | -13 ±3%^{**} | -18 ±3%* |

| | | | |
|---|---|---|---|
| Values are given as % decrease compared with sham operated control and expressed as means ± **SEM** (n=5-6) ^{**}p< 0,05, ^{**}p< 0,01 versus sham operated mice. | | | |

### Expression of osteoblast-and osteoclast associated genes

To investigate molecular mechanisms behind the bone phenotypic alterations in adult female ERβ -/- mice, the expression of osteoblast-α 1 (I) collagen (Col. I), alkaline phosphatase (ALP), osteocalcin (OC)) and osteoclast-(tartrate-resistant acid phosphatase (TRAP), cathepsin K (cat K))-associated mRNAs was assessed by RT-PCR of RNA extracted from long bones from wild type (wt) and ERβ -/- mice (Fig. 3). The expression levels of these genes were generally higher, and not sexually differentiated, in prepubertal compared to adult wt mice, consistent with increased bone metabolism and turnover during rapid post-natal growth. In female adult ERβ -/- mice, the expression of the osteoblast-associated genes was however increased compared to age-matched wt female mice. As to the osteoclast-associated genes, no difference could be observed between adult ERβ -/- and wt female mice, although expression levels were higher in adult females compared to males.

### Comparison between adult (11 wk.) and Senescent (1 year) mice.

### Various bone parameters were measured in 11 week old (i.e. Adult) and in older, one year old mice using the techniques described above and including conventional histomorphological examination. The results are shown in the Table 6.

**Table 6**

| TRABECULAR BONE VOLUME IN ADULT (11 wk) AND SENESCENT (1 YR) BERKO MICE | | | | | | |
|---|---|---|---|---|---|---|
| | Female | | | Male | | |
| | 11 wk | 1 yr | 1 yr/11wk | 11 wk | 1 yr | 1 yr/11 wk |
| Wild-type | 0.326 ±0.042 | 0.167 ±0.036 | 0.51 | 0.363±0.071 | 0.175 ±0.033 | 0.48 |
| BERKO | 0.345 ±0.071 | 0.278 ±0.106 | 0.80 | 0.363±0.070 | 0.147 ±0.028 | 0.40 |
| KO/WT | 1.06 | 1.67 | | 1.0 | 0.84 | |
| p | 0.55 | 0.04 | | 0.99 | 0.22 | |
| Expressed as BV/TV (%); Bone volume /Total volume (Histomorphometry) | | | | | | |

| TRABECULAR BONE DENSITY IN ADULT (11 wk) AND SENESCENT (1 YR) BERKO MICE | | | | | | |
|---|---|---|---|---|---|---|
| | Female | | | Male | | |
| | 11 wk | 1 yr | 1 yr/11wk | 11 wk | 1 yr | 1 yr/11 wk |
| Wild-type | 0.288 ±0.018 | 0.086 ±0.032 | **0.30** | 0.316±0.023 | 0.171 ±0.042 | 0.54 |
| BERKO | 0.275 ±0.027 | 0.159 ±0.067 | **0.58** | 0.309±0.030 | 0.146 ±0.030 | 0.47 |
| KO/WT | 0.95 | **1.85** | | 0.98 | 0.85 | |
| p | 0.31 | **0.01** | | 0.70 | 0.30 | |
| Expressed as mg/mm³ (pQCT) | | | | | | |

| CORTICAL BONE DENSITY IN ADULT (11 wk) AND SENESCENT (1 YR) BERKO MICE | | | | | | |
|---|---|---|---|---|---|---|
| | Female | | | Male | | |
| | 11 wk | 1 yr | 1 yr/11wk | 11 wk | 1 yr | 1 yr/11 wk |
| Wild-type | 1.147 ±0.028 | 1.224 ±0.041 | 1.067 | 1.156±0.045 | 1.213 ±0.047 | 1.049 |
| BERKO | 1.182 ±0.028 | 1.271 ±0.055 | 1.075 | 1.166±0.019 | 1.229 ±0.013 | 1.054 |
| KO/WT | **1.03** | **1.04** | | 1.01 | 1.01 | |
| P | **0.02** | **0.05** | | 0.68 | 0.46 | |
| Expressed as mg/mm³ (pQCT) | | | | | | |

| BODY WEIGHT IN ADULT (11 wk) AND SENESCENT (1 YR) BERKO MICE | | | | | | |
|---|---|---|---|---|---|---|
| | Female | | | Male | | |
| | 11 wk | 1 yr | 1 yr/11 wk | 11 wk | 1 yr | 1 yr/11 wk |
| Wild-type | 21.5±1.22 | 31.0±5.4 | 1.44 | 23.3±0.54 | 40.4±6.02 | 1.73 |
| BERKO | 25.2±2.92 | 30.2±5.6 | 1.20 | 25.4±1.63 | 34.5±5.6 | 1.36 |
| KO/WT | **1.17** | **0.97** | | 1.09 | 0.85 | |
| P | **0.01** | **0.79** | | 0.24 | 0.29 | |
| Expressed in grams | | | | | | |

| FAT CELL VOLUME IN THE TRABECULAR BONE AREA | | | | | | |
|---|---|---|---|---|---|---|
| | Female | | | Male | | |
| | 11 wk | 1 yr | 1 yr/11 wk | 11 wk | 1 yr | 1 yr/11 wk |
| Wild-type | | 38.9±23.7 | | | | |
| BERKO | | 15.3±13.7 | | | | |
| KO/WT | | 0.39 | | | | |
| P | | 0.06 | | | | |
| Expressed as fat cell volume/total tissue volume (%) | | | | | | |

From these results it can be seen that the loss of bone trabecular mass in older mice is slowed down in female BERKO mice compared with wild-type female mice. There is also a good correlation between the histomorphological analysis and the other measurement techniques indicating the reliability of the latter techniques. Accordingly, an ERβ antagonist may be used to slow the natural reduction in bone trabecular mass in aging female mammals such as humans.

The present study demonstrated that the adult female ERβ -/- mice had an increased total BMC caused by increased cortical BMC while the trabecular volumetric BMD was unchanged. This increase in cortical BMC was mainly caused by an increase in the cortical cross-sectional area, associated with a radial cortical growth (increased periosteal circumference) of the bones. No effect was seen in male ERβ -/- mice. Actually, the gender differences seen in adult wt mice, with higher total BMC, increased cross-sectional cortical area and increased periosteal circumference in males compared with females, were not seen in adult ERβ-/- mice. Thus, it appears that the cortical bone in female ERβ-/- mice displays a loss of feminization. It has previously been demonstrated that gonadectomy of young rats results in decreased radial cortical bone growth in males and increased radial growth in females such that the sex difference is largely eliminated. The sex difference is reestablished in ovariectomized (Ovx) rats by administration of estrogen to females (Turner *et al* (1994) *supra,* Turner, R.T., *et al* (1987) *J Bone Miner Res* **2**, 115-122). This finding together with our present results of an apparent loss of feminization of the cortical bone in adult female ERP -/- mice indicates that ERβ may be involved in feminization of the cortical bone in rodents. The effect of ERβ on cortical bone mass may include direct effects on the bone tissue or indirect effects including regulation of systemic hormones. A direct effect on the bone tissue is supported by recent findings that ERβ is expressed in osteoblasts (Onoe, Y., *et al* (1997) *Endocrinology* **138,** 4509-4512,Arts, J., Kuiper, G.G., *et al* (1997) *Endocrinology* **138,** 5067-5070,Vidal, O., *et al* (1999) *J Bone Miner Res **supra**). In* the tibial long bone, which has almost completed its longitudinal growth before the onset of puberty, an increase in BMC but not bone length was seen in the adult female ERβ -/- mice compared with wt mice. This finding demonstrates that the increased BMC in adult female ERβ -/- mice not only was caused by a stimulation of longitudinal bone growth.

It is well known from clinical and rat studies that Ovx results in a decrease in BMC and that this decrease predominantly is caused by a reduction of the trabecular volumetric BMD (Turner, R.T., *et al* (1994) *supra).* The Ovx mouse model is less well characterized. However, it was recently demonstrated that ovariaectomy in mice also results in a large decrease in trabecular volumetric BMD (Andersson, A., *et al* (1998) *Bone* **23**, S631). Interestingly, in the present study, no reduction in trabecular volumetric BMD was seen in ERβ -/- females compared with wt mice. These findings together indicate that Ovx-induced reduction in trabecular volumetric BMD is not solely dependent on ERβ. Therefore, one may speculate that either ERα by itself or that ERα and ERβ in a redundant manner protects against Ovx-induced trabecular bone loss. Future experiments with Ovx of ERα, ERβ and ERαβ double knockout mice will provide more insight to the receptor specificity of estrogens on bone mass. A functional receptor specificity for ERα versus ERβ in cardiovascular homeostatis has recently been described (Iafrati, M.D., *et al* (1997) *Nat Med* **3**, 545-548). It was demonstrated that estrogen inhibits vascular injury response by a mechanism that is independent of the ERα, suggesting that ERβ was involved in this response. Thus, ERα and ERβ may have both independent and common functions in normal physiology as well in pathophysiology.

In conclusion, ERβ -/- mice have unchanged trabecular BMD. In contrast, adult female mice devoid of ERβ have an increased cortical bone mineral content caused by an increased cross-sectional bone area, resulting in an apparent loss of feminization of the cortical bone. This finding indicates that ERβ is essential for the endogenous estrogen effect on cortical bone in female mice. The mechanism(s) of action for the effect of ERβ on cortical bone remains to be elucidated.

## Claims

1. A method of selecting compounds for the treatment or prevention of osteoporosis, the method comprising selecting a compound on the basis of its ability to antagonise agonist-dependent ERβ activity and wherein the effect of the compound on a bone-related parameter in a mammal is also determined, **characterised in that** the compound has a binding affinity for ERβ of less than 80nM.

2. A method according to claim 1 in which the compound has a binding affinity of less than 10nM for ERβ.

3. A method according to claim 1 or 2 in which the compound has a binding affinity of between 0.001 nM and 10nM for ERβ.

4. A method according to claim 1, 2 or 3 in which the compound has no ERα-antagonist activity.

5. A method according to claim 1, 2 or 3 in which the compound has negligible ERα-antagonist activity.

6. A method according to claim 5 in which the negligible ERα-antagonist activity is assessed by comparison of compound agonism with estradiol agonism, wherein greater than or equal to 50% agonism compared with estradiol is negligible ERα-antagonist activity.

7. A chemical compound determined by a method according to any one of claims 1 to 6.

8. An osteoporosis treatment or prevention composition comprising or consisting of an ERβ-selective antagonist.

9. An osteoporosis treatment or prevention composition comprising or consisting of an ERβ antagonist.

10. A pharmaceutical composition useful for treating or preventing osteoporosis comprising or consisting of an ERβ -selective antagonist.

11. An osteoporosis treatment or prevention composition according to claim 9 in which the ERβ- antagonist has no or negligible ERα- antagonist activity.

12. An osteoporosis treatment or prevention composition according to claim 8, 9, 10 or 11 in which the ERβ antagonist has a binding affinity of less than 80nM for ERβ.

13. An osteoporosis treatment or prevention composition according to claim 12 in which the ERβ antagonist has a binding affinity of less than 10nM for ERβ.

14. An osteoporosis treatment or prevention composition according to claim 13 in which the ERβ antagonist has a binding affinity of 0.001 to 10nm for ERβ.

15. An osteoporosis treatment or prevention composition according to any of claims 8 to 14 which includes an estrogen, an estrogen agonist or a SERM.

16. The use of an ERβ antagonist in the preparation of a pharmaceutical composition for the treatment or prevention of osteoporosis.

17. The use of an ERβ antagonist in the preparation of a pharmaceutical composition for the inhibition of bone resorption in an adult mammal.

18. The use of an ERβ antagonist in the preparation of a pharmaceutical composition for the reduction of bone resorption in an adult mammal.

19. The use of an ERβ antagonist in the preparation of a pharmaceutical composition for slowing a reduction in, or stabilising, or increasing cortical bone mass in an adult mammal.

20. The use of an ERβ antagonist in the preparation of a pharmaceutical composition for slowing a reduction in, or stabilising or increasing trabecular bone mass in an adult mammal.

21. The use of an ERβ antagonist in the preparation of a pharmaceutical composition for reducing the risk of bone fracture in an adult mammal.

22. A use according to any one of claims 16 to 21 in which the ERβ antagonist has no or negligible ERα antagonist activity.

23. A use according to any one of claims 16 to 22 in which the ERβ antagonist has a binding affinity of less than 80nM for ERβ.

24. A use according to claim 23 in which the ERβ antagonist has a binding affinity of less than 10nM for ERβ.

25. A use according to claim 24 in which the ERβ antagonist has a binding affinity of 0.001-10nM for ERβ.
